# EUROPEAN PATENT APPLICATION

(11) **EP 3 812 003 A1**
(43) Date of publication of application: **28.04.2021**
(21) Application number: 19211459.3
(22) Date of filing: 26.11.2019
(51) Int. Cl.: A61N 1/37, A61N 1/39, H03K 17/00

(54) **AN ELECTRIC CIRCUIT COMPRISING A THYRISTOR**

(30) Priority: 25.10.2019 US 201962925769 P
(71) Applicant: BIOTRONIK SE & Co. KG, 12359 Berlin (DE)
(72) Inventor: Porter, Matthew A., Molalla, Oregon 97038 (US); Benson, Jonathan M., West Linn, Oregon 97068 (US)
(74) Representative: Yang, Tian

(57) **Abstract**

An electric circuit (2) comprises a thyristor (T) having an anode (Al), a cathode (Kl) and a thyristor gate (Gl). The thyristor (T) in a blocking state blocks a current flow from the anode (Al) to the cathode (Kl) and in a conductive state is conductive for a current flow from the anode (Al) to the cathode (Kl), wherein the thyristor (T) is switchable from the blocking state to the conductive state by applying a current to the thyristor gate (Gl) exceeding a switching level. A pulse generator circuit (21) is configured to apply, in the process of switching the thyristor (T) from the blocking state to the conductive state, a current (P1) to the thyristor gate (G1) at a current level below the switching level normally required to switch the thyristor on.

## Description

The present invention generally relates to an electric circuit, to a medical device, in particular an implantable medical device having an electric circuit and to a method for operating an electric circuit.

Specifically, the present invention relates to an electric circuit comprising a thyristor having an anode, a cathode and a thyristor gate.

A thyristor, generally and as well-known in the art, is a solid-state semiconductor device having four layers of alternating P- and N-type materials with access to connect typically to the gate, anode and cathode structures. A thyristor acts as a switch which can be switched from a blocking state in which current from the anode to the cathode is blocked, to a conductive state in which a current can pass from the anode to the cathode. By injecting adequate current into the gate of the thyristor and initiating adequate current flow from anode to cathode, the thyristor can be switched from the blocking state to the conductive state and hence can be switched on such that a current can flow from the anode to the cathode.

An electric circuit of this kind may, for example, be used in an implantable medical device, in particular an implantable medical device having a defibrillator function, such as an implantable cardioverter defibrillator or a cardiac resynchronization therapy device. An implantable cardioverter defibrillator, for example, is configured to monitor the rate and duration of the heart electrical activity and to deliver an electric shock when the heart rate exceeds a threshold. The delivery of an electric shock herein involves the generation and injection of a high-energy electrical pulse, for example by way of a lead extending into the heart, for example into a ventricle of the heart, to provide a therapy for, for example, ventricular fibrillation.

When a thyristor within an electric circuit, for example in an implantable defibrillator, is switched from the blocking state to the conductive state and the thyristor is hence triggered to deliver electric energy to a load, in particular a capacitive and/or resistive load like the heart and surrounding body in the context of a defibrillation therapy, transient electrical signals resulting from the thyristor switching having a large voltage slope (dV/dt) and/or current slope (dI/dt) may arise and may interact, by way of electromagnetic interference, with connected or adjacent circuits of other circuitry of the implantable defibrillator. With a conventional activation of the thyristor, a rise to an output voltage on the load of, for example, 800 V may occur in a nanosecond range, for example within 100 nanoseconds, during which time the current may rise to 30 Amperes. Such transient signals may be capacitively coupled, inductively or magnetically coupled, or conducted into connected or adjacent circuits, making it necessary to apply measures to prevent an undesired coupling, to maintain signal integrity within the processing circuitry and to eliminate a potential voltage overstress in the implantable defibrillator. Such measures may include an adaptation of the circuit layout, a specific shielding, for example by including additional shielding planes in printed circuit boards, additional components such as capacitors, inductors or shunting semiconductors to block or bypass transient signals or filter components to filter out or alter transient signals.

There is a desire to provide an electric circuit making it possible to reduce the magnitude of the transient signals by switching the thyristor on in a controlled manner, for example in the context of a defibrillation function of an implantable medical device, so as to not need to employ the additional measures described above to prevent the effects of the transient signals.

US 5,852,556 describes an AC/DC power converter circuit having means for protecting thyristors against surge voltages. The converter comprises multiple light-triggered thyristors connected in series, which arrangement may exhibit a transient vulnerable phase starting immediately after switch off and ending when the forward withstand voltage recovers to a rated value. In order to retain the thyristor in a partially conductive state during such vulnerable transient, a gate signal consisting of single continuous sub-trigger threshold gate current pulse is supplied to the thyristors. But this addresses the self-protection aspect of the thyristors and is not a solution for the prevention of electrical interference with external circuitry.

The objective of the present invention is to provide an electric circuit, an implantable medical device comprising an electric circuit and a method for operating an electric circuit allowing for a controlled switching on of a thyristor in particular to reduce the amplitude of the transient electrical signals and thereby reduce the risk of their effects occurring during the switching.

This objective is addressed by means of an electric circuit comprising the features of claim 1.

In one aspect, an electric circuit comprises a thyristor having an anode, a cathode and a thyristor gate, wherein the thyristor in a blocking state blocks a current flow from the anode to the cathode and in a conductive state is conductive for a current flow from the anode to the cathode, wherein the thyristor is switchable from the blocking state to the conductive state by applying a current to the thyristor gate exceeding a switching threshold level. The electric circuit furthermore comprises a current generator circuit configured to apply, in the process of switching the thyristor from the blocking state to the conductive state, a current to the thyristor gate at a level below the switching threshold level.

For switching the thyristor on, i.e., to transfer the thyristor from the blocking state to the conductive state, generally a current exceeding a defined switching threshold level, typically specified by thyristor manufacturers as the parameter Gate Trigger Current, which is specific to thyristor type, must be injected into the thyristor gate. Once the thyristor has been switched on by a sufficient gate current exceeding the Gate Trigger Current threshold level, the thyristor generally remains latched in the conductive state, such that the current no longer needs to be supplied to the thyristor gate for the thyristor to remain in the conductive state.

Generally, if a trigger current is supplied to the gate above the Gate Trigger Current threshold level, the thyristor will substantially immediately switch on (after a propagation delay within a time range of a fraction of 1 microsecond to several microseconds, for example in about 1 microsecond), where, once the switching has begun, the switching signal exhibits transients of high dV/dt and dI/dt. To avoid transients involving an excessive dV/dt and dI/dt, it herein is proposed to switch the thyristor on using a current generator circuit configured to apply one or a series of multiple current pulses, which each by itself is not sufficient to transfer the thyristor from the blocking state to the conductive state, but may contribute to the switching by causing a low-level, partial conduction of the thyristor. Each current pulse has a current level below the nominally required switching level, such that the thyristor will not immediately switch and latch on when supplying a current pulse, but will enter into a partial conduction state while each pulse is applied to build up voltage on the load circuit when applying one pulse after another.

Another important aspect of thyristor switching is that, not only is the Gate Trigger Current level important as to whether the thyristor switches immediately from blocking to conducting, but also whether the magnitude of current from anode to cathode has exceeded another characteristic parameter of the thyristor called Latching Current. Latching Current is the minimum current from thyristor anode to cathode that causes the internal thyristor structure to bias such that the current is sustained without further injection of gate trigger current. It is specific to each thyristor and specified by the thyristor manufacturer. In order to achieve reduction of high dV/dt and dI/dt transient signals during thyristor switching, the load through which the thyristor is switching current must at least temporarily be high enough impedance that when the thyristor conducts, the current from anode to cathode does not exceed the Latching Current level. This means that if the load circuit would normally be such low impedance that the current switched through the thyristor would exceed the Latching Current, then the impedance of the load circuit must be temporarily increased sufficiently to reduce the thyristor current below the Latching Current.

That each gate current pulse has a current level below the switching level is, in the context of the present text, to be understood to express that each current pulse by itself is not suitable to switch the thyristor from its blocking state to the conductive state. This can be achieved by appropriately designing the current pulses such that they are below a nominal trigger current threshold. According to an embodiment of the present invention which is configured using a thyristor referred to as a Anode Gated Thyristor with the load connected to the cathode, the load impedance must be high enough so that the cathode voltage is able to rise as a result of the partial conduction of the thyristor in response to the low level gate current. Larger capacitance loads require more pulses of the nominal current pulse to achieve final desired output voltage. Alternatively or in addition, during an initial development phase or during actual operation the effect a current pulse has to the output voltage across a load circuit connected to the thyristor can be monitored. In particular, the output voltage can be monitored to ensure that with each current pulse only a fraction of the anode voltage, i.e. a voltage of a supply voltage source supplied to the anode of the thyristor, is transferred to the load circuit to contribute to the output voltage.

In one embodiment, the pulse generator circuit is configured to apply, in the process of switching the thyristor from the blocking state to the conductive state, a series of current pulses to the thyristor gate, each current pulse having a current level below the Gate Trigger Current switching level and the Latching Current level. By controlling the pulse width and the duty cycle of the series of current pulses, the pulse generator circuit may control the time duration over which the thyristor transfers voltage to the load such that a rate of a voltage and current rise may be controlled to not exceed an undesired level. Each current pulse by itself does not cause the thyristor to switch on because the Gate Trigger Current and Latching Current threshold levels are not exceeded, however, the series of current pulses together cause the thyristor to progressively increase the voltage on the load. At a point that the voltage on the load is sufficiently increased, such that the magnitude of the transients (dV/dt and dI/dt) would be acceptably low, the thyristor is finally switched on by applying a current that is above the Gate Trigger Current threshold level, and the differential voltage that it switches to the load is significantly lower than the full voltage so that the overall dV/dt and dI/dt are reduced.

The pulse width used for the current pulses may be constant for the series of current pulses or may vary over the series of current pulses. The pulse width may, for example, range between 100 nanoseconds and 10 µs, for example between 500 nanoseconds and 1 µs.

A series of current pulses used to switch the thyristor on may, for example include a number of current pulse anywhere between 5 and 300, for example anywhere between 10 and 20 current pulses, for example 12 current pulses.

The duty cycle of the series, i.e. the ratio between the pulse width and the period of the series (which is the inverse of the frequency of the current pulses) may, for example lie between 5% and 80%, for example between 10% and 50%.

The current of the pulses in the series which are less than the Gate Trigger Current provided to the gate of the thyristor may for example lie between 1 milliamp and 20 milliamps, for example between 5 milliamps and 10 milliamps

In one embodiment, the electric circuit comprises a load circuit for outputting an output voltage, for example to an electrode lead of an implantable medical device having a defibrillation function. Such load circuit may, for example be connected to the cathode of the thyristor, whereas the anode of the thyristor is connected to a supply voltage source. The load circuit may, for example comprise a load resistor and/or a load capacitor across which the output voltage is provided, wherein in the conductive state, the output voltage may substantially match the supply voltage applied to the anode of the thyristor.

Herein, in the process of switching the thyristor from the blocking state to the conductive state, by applying a series of current pulses that are less than the Gate Trigger Current threshold to the thyristor gate, the output voltage provided at the load circuit may be caused to progressively build up towards the supply voltage applied at the anode of the thyristor, the rate of increase of the output voltage being determined by the characteristics of the series of current pulses, i.e. the pulse width, duty cycle, frequency and amplitude of the current pulses injected into the gate during the process of switching the thyristor on.

In one embodiment, the pulse generator circuit comprises a switching element switchable between an off-state and an on-state for supplying a current pulse to the thyristor gate. The switching element may in particular be a transistor, in particular a MOSFET transistor, having a gate connected to a control pulse generation circuit and a drain connected to the thyristor gate. At its source the transistor may in particular be connected to a control voltage such that by switching the transistor from its off-state to its on-state, a controlled current may be caused to flow through the transistor and may be supplied as a current pulse to the gate of the thyristor connected to the drain of the transistor.

The control pulse generation circuit may be configured to apply one or multiple voltage pulses to the gate of the transistor. In particular, the control pulse generation circuit may apply a series of voltage pulses to the transistor gate, such series of voltage pulses causing a series of current pulses to be injected into the gate of the thyristor, such that the thyristor is caused to progressively transfer from the blocking state to the conductive state in a controlled manner, the pulse width, duty cycle and frequency of the series of current pulses being controlled by the switching of the transistor according to the series of voltage pulses.

An implantable medical device comprising an electric circuit of the kind described above may in particular be an implantable defibrillator, for example an implantable cardioverter defibrillator (ICD) or a cardiac resynchronization therapy (CRT) device.

The objective is also addressed by means of a method for operating an electric circuit, the electric circuit comprising a thyristor having an anode, a cathode and a thyristor gate, wherein the thyristor in a blocking state blocks a current flow from the anode to the cathode and in a conductive state is conductive for a current flow from the anode to the cathode, wherein the thyristor is switchable from the blocking state to the conductive state by applying a current to the thyristor gate exceeding a switching level, the method including: applying, using a pulse generator circuit, in the process of switching the thyristor from the blocking state to the conductive state a current pulse to the thyristor gate at a current level below the switching level.

According to another embodiment of the present invention, the thyristor is a silicon controlled rectifier (SCR). An SCR is constructed such that the anode would be connected to the low side of the load and the cathode is connected to ground. All other aspects of the low level gate current switching still apply.

According to an embodiment of the present invention, other switches of similar behavior like a thyristor can be applied in place of the thyristor. Examples are triacs or gate turn-off thyristors (GTO).

The advantages and advantageous embodiments described above for the electric circuit equally apply also to the method such that it shall be referred to the above in this respect.

The various features and advantages of the present invention may be more readily understood with reference to the following detailed description and the embodiments shown in the drawings. Herein,
- Fig. 1: shows a schematic drawing of an implantable medical device in the form of an implantable defibrillator, in an implanted state;
- Fig. 2: shows a schematic view of the implantable medical device;
- Fig. 3: shows a schematic view of an electric circuit, comprising a thyristor and a load circuit being fed by the thyristor;
- Fig. 4: shows a series of current pulses to be injected into a gate of the thyristor of the electric circuit of Fig. 3 to progressively transfer the thyristor from a blocking state to a conductive state;
- Fig. 5: shows a schematic view of the electric circuit together with a pulse generator circuit for injecting current pulses into the thyristor;
- Fig. 6: shows a series of voltage pulses for controlling a switching element in the form of a transistor of the pulse generator circuit;
- Fig. 7: shows a series of pulses for switching the thyristor on and a resulting output voltage of the electric circuit;
- Fig. 8: shows an enlarged view of a voltage pulse applied to the pulse generator circuit and a corresponding current pulse injected into the thyristor; and
- Fig. 9: shows a subset of pulses of the series of Fig. 7.

Subsequently, embodiments of the invention shall be described in detail with reference to the drawings. In the drawings, like reference numerals designate like structural elements.

It is to be noted that the embodiments are not limiting for the invention, but merely represent illustrative examples.

The present invention relates to an electric circuit comprising a thyristor, in particular to an electric circuit to be used within an implantable medical device, such as an implantable medical device having a defibrillation function, for example an implantable cardioverter defibrillator or a cardiac resynchronization therapy device.

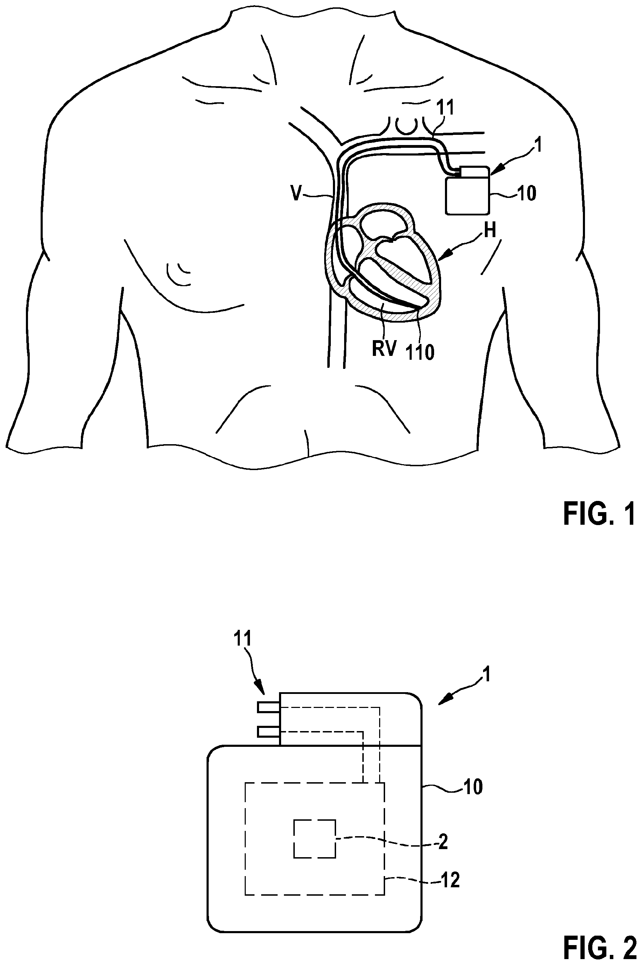

Referring now to Fig. 1, an implantable medical device 1 in the form of an implantable defibrillator, for example an implantable cardioverter defibrillator or a cardiac resynchronization therapy device, is implanted such that a generator device 10, for example rests subcutaneously in the region of the chest of a patient and leads 11 extend from the generator device 10 through the superior vena cava V, for example into the right ventricle RV of the patient's heart H. An electrode arrangement 110, for example in the vicinity of the tip of the leads 11 couples with cardiac tissue in order to inject stimulation energy into the human heart H to provide a defibrillation therapy.

Within a defibrillation function an electric shock at a high voltage (for example at around 800 V) and high energy is injected into a patient. Such energy is output as a high-energy pulse to provide for an electric shock to counteract a fibrillation of the heart.

For this, the generator device 10, as illustrated in Fig. 2, comprises an electric circuit 2 serving to output a high-energy, high-voltage electrical pulse to leads 11 connected to the generator device 10. The electric circuit 2 is controlled by a processing circuitry 12 of the generator device 10, the processing circuitry 12 including electronic components such as a processor and a communication circuitry to communicate with external systems, and an energy storage in the form of a battery.

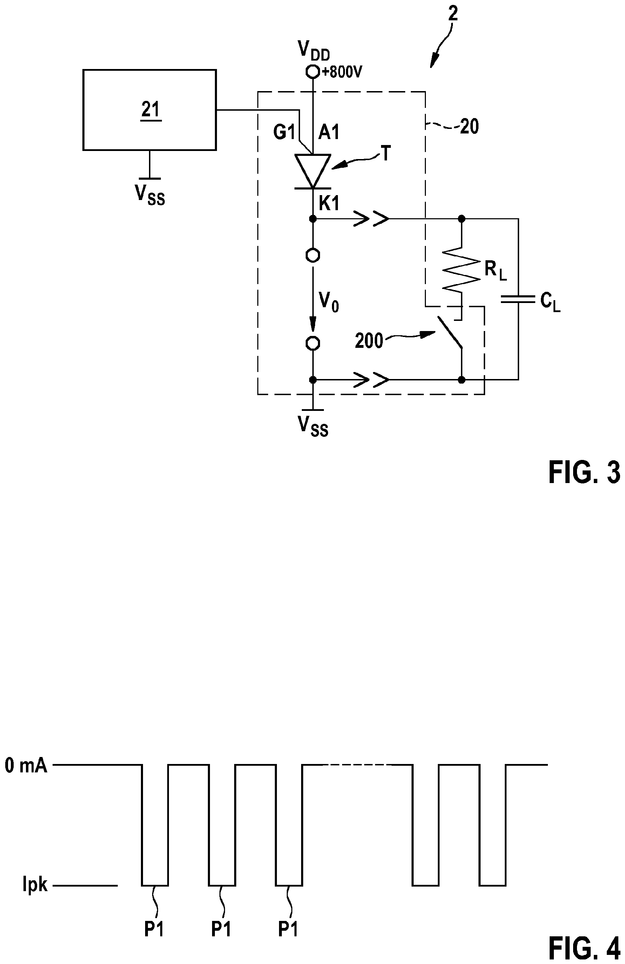

Referring now to Fig. 3, an electric circuit 2 as used, for example within an implantable medical device 1 having a defibrillation function comprises a thyristor T having an anode A1, a cathode K1 and a thyristor gate G1. A supply voltage source V_{DD} is connected to the anode A1, and a means of connecting a load circuit 200 is connected to the cathode K1 of the thyristor T. The load circuit 200 comprises a load resistor RL, which in the case of a ICD or CRT device represents the heart H, and a load capacitor CL which in the case of an ICD or CRT device represents the bulk capacitance of the heart H and surrounding body tissue as well as capacitors connected for other circuit functions, connected in parallel, the load resistor RL and load capacitor CL at one end being connected to the means for connecting to the cathode K1 of the thyristor T and at the other end being connected to a system ground V_{SS}. Across the load resistor RL and the load capacitor CL of the load circuit 200 an output voltage V₀ is provided.

The thyristor T and the load circuit 200 together form a thyristor circuit 20, within which the thyristor T can be switched between a blocking state and a conductive state. In the blocking state the thyristor T is non-conductive such that no currents are allowed to pass through the thyristor T, and the voltage V₀ across the load circuit 200 is substantially 0. In the conductive state, in turn, a current flows from the anode A1 to the cathode K1, such that the potential of the voltage source V_{DD} is passed towards the load circuit 200, and an output voltage V₀ is provided across the load circuit 200 substantially matching the voltage of the voltage source V_{DD}, for example 800 V.

For providing a high-energy pulse to trigger an electric shock the thyristor T is switched on, i.e. the thyristor T is transferred from the blocking state to the conductive state in order to switch the output voltage V₀ to the voltage of the supply voltage source V_{DD}. For transferring the thyristor T from the blocking state to the conductive state, herein, a trigger current is injected into the gate G1 of the thyristor T, such trigger current causing the thyristor T to become conductive, wherein the thyristor T remains latched in the conductive state once the thyristor T is switched on.

Generally, a trigger current above the Gate Trigger Current threshold level is required to switch the thyristor T on.

Generally, within a conventional thyristor circuit a thyristor T is switched on, after an inherent delay, substantially immediately, i.e. within nanoseconds, for example 100 ns, upon injecting a sufficient current into the gate G1 of the thyristor T. Such sudden transferring of the thyristor T from the blocking state into the conductive state may cause a rise of the output voltage V₀ at a significant rate (dV/dt), and likewise the slope of the current rise (dI/dt) will be substantial.

In order to control the switching time of the thyristor T within the electric circuit 2, it herein is proposed to use a pulse generator circuit 21 to inject a current into the gate G1 in a particular fashion in order to limit transient signals due to a voltage and current rise during the switching of the thyristor T from the blocking state into the conductive state to a level which is tolerable and in particular prevents an excessive coupling of the transient signals into adjacent circuitry, in particular the processing circuitry 12 of the implantable medical device 1.

Referring now to Fig. 4, in one aspect the pulse generator circuit 21 is used to inject a series of current pulses P1 into the gate G1 of the thyristor T, each current pulse P1 having an amplitude below the Gate Trigger Current level of the thyristor T such that each current pulse P1 by itself is not sufficient to switch the thyristor T on, but only contributes to induce a low-level conduction of the thyristor T, which progressively builds up over the series of current pulses P1 until, at the end of the series of current pulses P1, the thyristor T is switched on and remains latched in its conductive state.

The pulse generator circuit 21 in particular controls a pulse width, amplitude and frequency of the current pulses PI, wherein the current pulses PI, as illustrated in Fig. 4, each may have the same amplitude and pulse width, or may vary in their amplitude and pulse width over the series of current pulses P1.

At the same time, the load circuit is temporarily modified if necessary by inserting a high impedance, for example the open switch shown in Figure 3, in series such that while the gate current pulses are partially turning the thyristor on, the current from anode to cathode is maintained below the Latching Current threshold level. This allows the voltage on the load to not be drained off while it is advancing toward a high level.

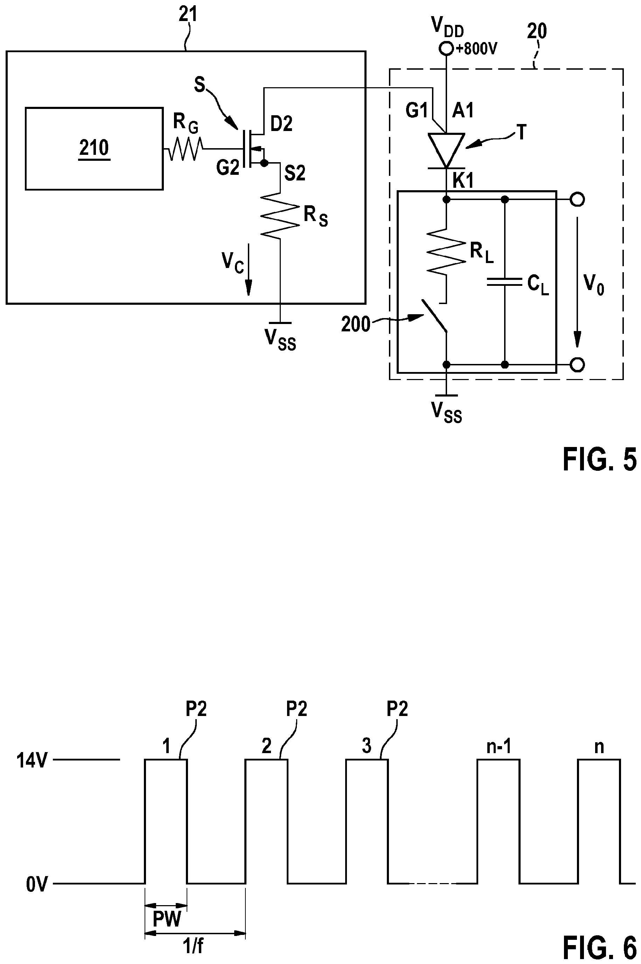

Referring now to Fig. 5, the pulse generator circuit 21, in one embodiment, comprises a switching element S in the form of a transistor, in particular a MOSFET transistor having a source S2, a drain D2 and a gate G2. The switching element S operates as a controlled current source Ic by connecting the source S2 to V_{SS} through a resistor RS. The drain D2 of the switching element S in the form of the transistor is connected either directly or through an impedance to the gate G1 of the thyristor T. The gate G2 of the switching element S in the form of the transistor is, via a resistor RG, connected to control pulse generation circuit 210 which serves to inject control voltage pulses P2, as illustrated in Fig. 6, into the gate G2 of the switching element S in the form of the transistor.

The control voltage pulses P2, as illustrated in Fig. 6, have a defined pulse width PW and are applied to the gate G2 of the switching element S in the form of the transistor at a defined frequency f. By means of the series of control voltage pulses P2, herein, the switching element S is periodically switched (at a period equal to 1/f) into a state of conduction such that current pulses P1, as illustrated in Fig. 4, are injected into the gate G1 of the thyristor T corresponding to the control voltage pulses P2 applied to the gate G2 of the switching element S in the form of the transistor.

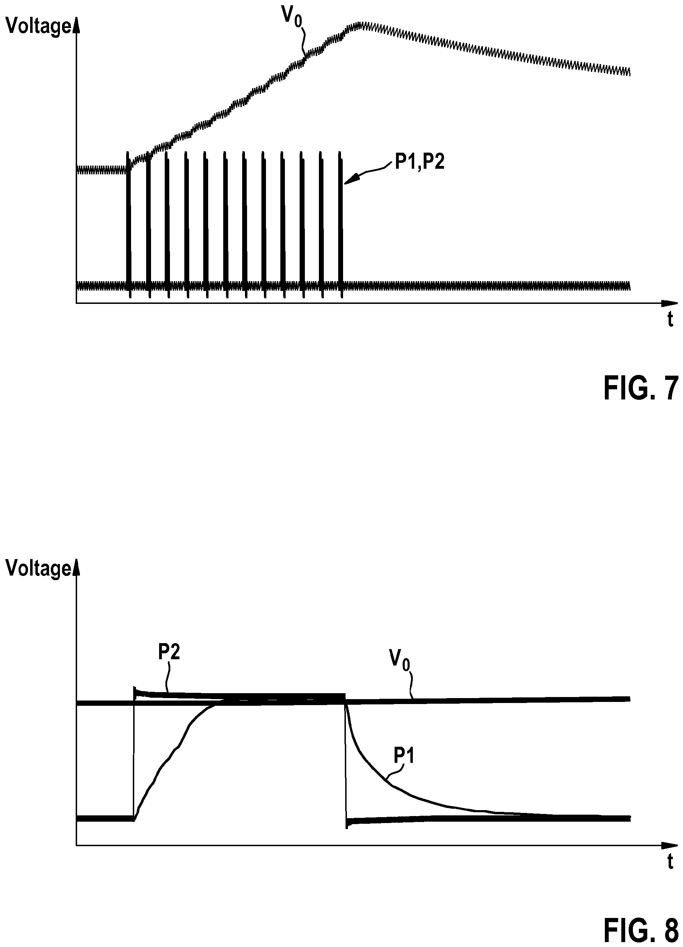

Referring now to Fig. 7, the series of control voltage pulses P2 applied to the switching element S in the form of the transistor of the pulse generator circuit 21 and the corresponding current pulses P1 injected into the gate G1 of the thyristor T cause a level of conduction at the thyristor T that does not rise to the level required to latch the thyristor on but are enough to progressively increase the output voltage on the load circuit, as shown in Fig. 7 according to the curve of the output voltage V₀ at the load circuit 200 connected to the cathode K1 of the thyristor T. With each pulse PI, P2, a fraction of the output voltage V₀ is transferred across the thyristor T towards the load circuit 200, such that the output voltage V₀ progressively builds up towards the voltage potential of the voltage source V_{DD} connected to the anode A1 of the thyristor T.

Each current pulse P1 is set, in its amplitude and pulse width, such that it does not exceed the nominal gate trigger current or latching current of the thyristor T. This can be achieved by appropriate settings of the pulse characteristics of the current pulses PI, controlled by the control voltage pulses P2 supplied by the control pulse generation circuit 210 of the pulse generator circuit 21. Alternatively or in addition, during development of the medical device 1 or during actual operation it can be monitored which effect each pulse PI, P2 has to the output voltage V₀ across the load circuit 200. In particular, the output voltage V₀ can be monitored to ensure that with each pulse PI, P2 only a fraction of the anode voltage, i.e. the voltage of the supply voltage source V_{DD} supplied to the anode A1 of the thyristor T, is transferred to the load circuit 200 to contribute to the output voltage V₀.

As the thyristor T is switched according to the series of current pulses P1 applied to the gate G1 of the thyristor T, the time for transferring the thyristor T from the blocking state to the conductive state can be controlled by the characteristics of the series of current pulses P1. In particular, by controlling the amplitude, pulse width and duty cycle of the series of current pulses PI, the duration for switching the thyristor T from the blocking state to the conductive state can be set such that transient signals arising during the switching are reduced in magnitude, or even substantially eliminated, and therefore cannot couple into other circuitry of the implantable medical device 1, such that signal integrity is maintained and a voltage overstress is avoided.

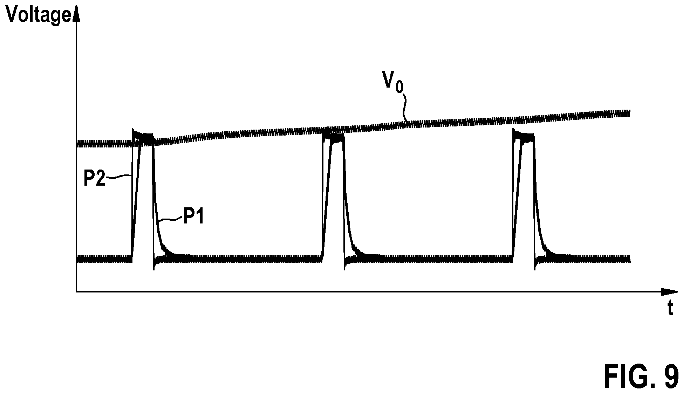

Referring now to Figs. 8 and 9, a control voltage pulse P2 at the gate G2 of the switching element S in the form of the transistor of the pulse generator circuit 21 has a defined, substantially rectangular shape. A current pulse P1 caused by the control voltage pulse P2 substantially corresponds to the control voltage pulse P2 and represents a current injected into the gate G1 of the thyristor T, causing it to contribute to a partial, low-level conduction of the thyristor T. With the load at least temporarily changed to a high impedance the voltage on the load progressively increases toward the full source voltage.

In an example embodiment, the resistor RG may have a value of 15 kΩ.

Each control voltage pulse P2 may have a pulse amplitude, for example between 5V and 15V, for example about 13 V.

The frequency f of the series of control voltage pulse P2 may lie between 1kHz and 1MHz, for example between 50kHz and 500kHz, for example at 200 kHz.

A number anywhere between 3 and 300 or more pulses, for example 12 pulses, may be used to cause the thyristor T to conduct current below its latching current level to slowly increase the voltage Vo on the load before the thyristor is triggered to switch from the blocking state to the conductive state.

A duty cycle of the series of pulses may range between 5% and 95%, for example 20%.

The pulse period may, for example, lie between 1 µs and 100 µs, for example 5 µs.

By means of applying a suitable series of pulses PI, P2 the time duration for switching the thyristor on may, for example lie in between 50 µs and 1000 µs, for example in between 150 µs and 300 µs. According to a preferred embodiment, the time duration for switching the thyristor may be approximately 200 µs.

An approach to switch a thyristor on by employing sub-trigger level current pulses may in particular be effective for high-impedance or high resistance series or parallel capacitive loads. It however could also be implemented in circuits with low-resistance loads where the thyristor, for example does not serve as primary current switch, such as an H-bridge configuration or totem-pole output, where the thyristor can first switch into a high-resistance load and a second inherently slower switch performs a current power switching.

### LIST OF REFERENCE NUMERALS

- 1: Implantable medical device
- 10: Generator device
- 11: Lead
- 110: Electrode arrangement
- 12: Processing circuitry
- 2: Electric circuit
- 20: Thyristor circuit
- 200: Load circuit
- 21: Pulse generator circuit
- 210: Control pulse generation circuit
- A1: Anode
- CL: Capacitor
- D2: Drain
- G1: Thyristor gate
- G2: Transistor gate
- H: Heart
- K1: Cathode
- P1: Current pulses
- P2: Voltage pulses
- PW: Pulse width
- RG, RL, RS: Resistor
- RV: Right ventricle
- S: Switching element (transistor)
- S2: Source
- T: Thyristor
- V: Superior vena cava
- V₀: Output voltage
- V_{C}: Control voltage
- V_{DD}: Supply voltage
- V_{SS}: Mass potential (ground)

## Claims

1. An electric circuit (2), comprising:
a thyristor (T) having an anode (A1), a cathode (K1) and a thyristor gate (G1), wherein the thyristor (T) in a blocking state blocks a current flow from the anode (A1) to the cathode (K1) and in a conductive state is conductive for a current flow from the anode (A1) to the cathode (K1), wherein the thyristor (T) is switchable from the blocking state to the conductive state by applying a current to the thyristor gate (G1) exceeding a switching level; and
a current generator circuit (21) configured to apply, in the process of switching the thyristor (T) from the blocking state to the conductive state, a current (P1) to the thyristor gate (G1) at a current level below the switching level.

2. The electric circuit (2) of claim 1, wherein the current generator circuit (21) is configured to apply, in the process of switching the thyristor (T) from the blocking state to the conductive state, a series of current pulses (P1) to the thyristor gate (G1), each current pulse (P1) having a current level below the switching level.

3. The electric circuit (2) of claim 2, wherein the pulse generator circuit (21) is configured to control a time duration for switching the thyristor (T) from the blocking state to the conductive state according to a pulse width (PW) and a duty cycle of the series of current pulses (P1).

4. The electric circuit (2) of one of claims 1 to 3, comprising a means for outputting an output voltage (V₀) to a load circuit (200).

5. The electric circuit (2) of claim 4 wherein the thyristor (21) at the anode (A1) is connected to a supply voltage (V_{DD}) and at the cathode (K1) is connected to a load circuit (200).

6. The electric circuit (2) of claim 5, wherein in the conductive state the output voltage (V₀) of the load circuit (200) substantially matches the supply voltage (V_{DD}).

7. The electric circuit (2) of one of claims 4 to 6, wherein in the process of switching the thyristor (T) from the blocking state to the conductive state the output voltage (V₀) is progressively increased by applying a series of current pulses (P1) to the thyristor gate (G1).

8. The electric circuit (2) of one of the preceding claims, wherein the pulse generator circuit (21) comprises a switching element (S) switchable between an off-state and an on-state for supplying a current pulse (P1) to the thyristor gate (G1).

9. The electric circuit (2) of claim 8, wherein the switching element (S) is a transistor having a transistor gate (G2) connected to a control pulse generation circuit (210).

10. The electric circuit (2) of claim 9, wherein the switching element (S) is a MOSFET transistor having a drain connected to the thyristor gate (G1).

11. The electric circuit (2) of claim 9 or 10, wherein the control pulse generation circuit (210) is configured to apply a voltage pulse (P2) to the transistor gate (G2) of the transistor.

12. An implantable medical device (1), comprising an electric circuit (2) of one of the preceding claims.

13. The implantable medical device (1) of claim 12, wherein the implantable medical device (1) is an implantable defibrillator.

14. A method for operating an electric circuit (2), the electric circuit (2) comprising a thyristor (T) having an anode (A1), a cathode (K1) and a thyristor gate (G1), wherein the thyristor (T) in a blocking state blocks a current flow from the anode (A1) to the cathode (K1) and in a conductive state is conductive for a current flow from the anode (A1) to the cathode (K1), wherein the thyristor (T) is switchable from the blocking state to the conductive state by applying a current to the thyristor gate (G1) exceeding a switching level, the method including:
applying, using a pulse generator circuit (21), in the process of switching the thyristor (T) from the blocking state to the conductive state a current pulse (P1) to the thyristor gate (G1) at a current level below the switching level.

15. Method according to claim 14, the method further includes: the current generator circuit (21) applies, in the process of switching the thyristor (T) from the blocking state to the conductive state, a series of current pulses (P1) to the thyristor gate (G1), each current pulse (P1) having a current level below the switching level.
